# EUROPEAN PATENT APPLICATION

(11) **EP 4 751 708 A1**
(43) Date of publication of application: **03.06.2026**
(21) Application number: 25218228.2
(22) Date of filing: 25.11.2025
(51) Int. Cl.: A61K 8/34, A61K 8/37, A61K 8/39, A61K 8/42, A61Q 5/10

(54) **MIXTURE OF SURFACTANTS**

(30) Priority: 28.11.2024 EP 24216288
(71) Applicant: Wella Germany GmbH, 64295 Darmstadt (DE)
(72) Inventor: Sitterberg-Muehlthau, Stephanie, 64295 Darmstadt (DE); Braun, Petra, 64295 Darmstadt (DE); Gross, Andrej, 64295 Darmstadt (DE); Godfrey, Simon Paul, 64295 Darmstadt (DE)
(74) Representative: Ziebig Hengelhaupt Intellectual Property Attorneys Patentanwaltskanzlei PartGmbB

(57) **Abstract**

The presently claimed invention relates to a mixture (I) comprising at least four surfactants (11) to (14), wherein the mixture (I) comprises 5 to 15 wt.% of at least one first non-ionic surfactant (11) having an HLB value in the range of 1 to 9, 45 to 65 wt.% of at least one second non-ionic surfactant (12) having an HLB value in the range of higher than 9 to 14, 10 to 30 wt.% of at least one third non-ionic surfactant (13) having an HLB value in the range of higher than 14 to 20, and 10 to 30 wt.% of at least one anionic surfactant (14) having an HLB value of higher than 17, wherein the % by weight values in each case are based on the total weight of the surfactants (11), (12), (13) and (14).

## Description

### Field of the invention

The presently claimed invention relates to a mixture (I) comprising at least four surfactants (I1) to (I4), wherein the mixture (I) comprises 5 to 15 wt.% of at least one first non-ionic surfactant (I1) having an HLB value in the range of 1 to 9, 45 to 65 wt.% of at least one second non-ionic surfactant (I2) having an HLB value in the range of higher than 9 to 14, 10 to 30 wt.% of at least one third non-ionic surfactant (I3) having an HLB value in the range of higher than 14 to 20, and 10 to 30 wt.% of at least one anionic surfactant (I4) having an HLB value of higher than 17, wherein the % by weight values in each case are based on the total weight of the surfactants (I1), (I2), (I3) and (I4).

### BACKGROUND OF THE INVENTION

Oxidative hair colouring has been a well-established technique for many years. Consumers seek the ability to achieve a broad spectrum of hair shades, while maintaining a pleasant and safe application experience. This necessitates achieving a high level of hair lift (removal of natural hair colour) to enable the creation of diverse shades, with minimal or acceptable hair damage, reduced or no ammonia odor during application, and minimal scalp irritation. In order to deliver a wide range of shades, a large number of dye precursors are used, and they need to be used at sufficient levels to achieve the desired shades.

Ammonia is commonly used in hair colour formulations to achieve high lift, enabling the creation of a wide range of dye shades. However, this high lift process often results in significant hair damage. Such damage manifests as dryness, reduced elasticity, brittleness, split ends, dullness, a matte appearance, decreased fullness, a rough surface, and reduced mechanical strength. Additionally, the use of ammonia is associated with its strong and unpleasant odour during application.

Monoethanolamine is frequently used in low-odour hair colour formulations as an alternative to ammonia. While it provides an acceptable level of hair lift with comparatively lower odour, it has its own limitations. Monoethanolamine tends to produce high levels of unwanted dye/alkali adducts, which can restrict the ability to achieve a broad range of dye shades.

Permanent and demi-permanent oxidative hair colouring products have been widely used in both professional salons and in home applications for decades. These products typically consist of two components: a tint composition and an oxidizing composition, which are packaged separately and mixed immediately before use to create the final colouring composition. The tint composition contains so-called oxidative dye precursors which are small molecules (typically oxidative primary dye precursors and oxidative coupler dye precursors) and an alkalizing agent, usually ammonia or an amine. These oxidative primary dye precursors react with the oxidative coupler dye precursors in the presence of an oxidizing agent to form larger, coloured dye products within the hair. The oxidizing composition contains the oxidizing agent to enable the colouring process. The oxidizing compositions typically contain a stabilized form of hydrogen peroxide and are sometimes referred to as the developer compositions.

The tint composition is thus mixed with the oxidizing composition immediately prior to the application to the hair to be coloured and the resulting mixture of the colouring composition is applied and left on the hair usually for 5 to 50 minutes before rinsing. The mixed colouring composition usually has an alkaline pH between about 8.5 and 10.5.

Oxidative hair colouring compositions that include ammonia or an amine as the alkalizing agent in the tint composition are often referred to as permanent hair colourants, also known as "Level 3" hair colourants. These products are marketed under brands such as Koleston Perfect^{®} by Wella Professionals in Europe. In these formulations, hydrogen peroxide, especially in the presence of an alkalizing agent, not only facilitates the oxidation of dye precursors but also bleaches melanin, enabling the creation of shades that are lighter or darker than the natural hair colour.

Demi-permanent hair colourants, commonly referred to as "Level 2" hair colourants, are also marketed as two-component systems. Like permanent hair colourants, they utilize oxidative primary dye precursors and oxidative coupler dye precursors. However, they differ in the choice of alkalizing agents, favouring alternatives to ammonia, such as monoethanolamine (MEA) or aminomethylpropanol (AMP; 2-amino-2-methylpropan-1-ol). Additionally, demi-permanent formulations typically operate with lower concentrations of hydrogen peroxide, usually 1 to 3 wt.% in the mixed colouring composition, compared to 3 to 6 wt.% or higher in permanent Level 3 products. In cases where more significant lightening or colour lift is desired, hydrogen peroxide levels may reach up to 8 wt.% for specific shades.

Demi-permanent hair colourants usually cause less melanin bleaching and thus less lift of natural hair colour. The resulting dyes may also penetrate less deeply in the hair shaft so that demi-permanent hair colourants can be less long lasting than permanent hair colourants. On the other hand, demi-permanent hair colourants are usually also less damaging to the hair structure than permanent hair colourants and the resulting hair colour may also be more natural looking. A further advantage is that demi-permanent hair colouring compositions do not have the strong ammonia smell of permanent hair colouring compositions and thus have a better consumer experience. A professional brand of demi-permanent hair colourants in Europe is for example Colour Touch^{®} from Wella Professionals in Europe. The mixed colouring composition usually has an alkaline pH of between about 8.5 and 10.5.

The oxidizing agents and oxidative dye precursors used in these formulations are typically incorporated into a "chassis formulation". This chassis includes the alkalizing agent and other components, such as cosmetically acceptable aqueous or aqueous-organic media, fatty substances, surfactants, chelants, thickeners, antioxidants, and fragrances, to create a comprehensive and effective hair colouring system.

As previously mentioned, it is crucial to avoid scalp irritation during oxidative hair colouring. The presence of a significant amount of residual highly mobile aqueous phase in the chassis formulation increases the likelihood of scalp irritation. Accordingly, there is a need for an oxidative hair colouring chassis that facilitates the creation of a full range of desired colour shades and intensities, delivers high levels of lift, and ensures scalp protection throughout the colouring process.

### SUMMARY OF THE INVENTION

It is an object of the presently claimed invention to provide an oxidative hair colouring chassis that facilitates the creation of a full range of desired colour shades and intensities, delivers high levels of lift, and ensures scalp protection throughout the colouring process.

The object has been solved by a mixture (I) comprising at least four surfactants (I1) to (I4), wherein the mixture (I) comprises
(I1) 5 to 15 wt.% of at least one first non-ionic surfactant having an HLB value in the range of 1 to 9,
(I2) 45 to 65 wt.% of at least one second non-ionic surfactant having an HLB value in the range of higher than 9 to 14,
(I3) 10 to 30 wt.% of at least one third non-ionic surfactant having an HLB value in the range of higher than 14 to 20,
(I4) 10 to 30 wt.% of at least one anionic surfactant having an HLB value of higher than 17,
wherein the % by weight values in each case are based on the total weight of the surfactants (I1), (I2), (I3) and (I4).

In another aspect, the presently claimed invention is directed to a kit comprising
(i) a tint composition comprising components (A), (B), (C), (D) and the inventive mixture (I), and
(ii) an oxidative composition comprising component (E) as defined in the present invention.

In yet another aspect, the presently claimed invention is directed to a method of treating hair comprising the steps of:
a. providing the tint composition (i) comprising components (A), (B), (C), (D) and a mixture (I) as defined in the present invention,
b. providing an oxidative composition (ii) comprising component (E) as defined in the present invention,
c. mixing the tint composition (i) and the oxidative composition (ii) to obtain a mixed oxidative hair colouring composition according to the present invention,
d. applying the oxidative hair colouring composition onto the hair;
e. leaving the oxidative hair colouring composition on the hair for a period in the range from ≥ 5 to ≤ 50 minutes; and
f. subsequently rinsing the oxidative hair colouring composition from the hair.

Surprisingly, it was discovered that the use of an inventive mixture (I), comprising non-ionic surfactants with distinct HLB values spanning low (1 to 9), medium (higher than 9 to 14), and high (higher than 14 to 20) ranges, in combination with an anionic surfactant (HLB higher than 17), effectively protects the scalp and enhances lightening during oxidative hair colouring when included in an oxidative hair colouring chassis and/or composition.

Without being bound to a theory, it is assumed that any irritants to the scalp are especially comprised in any residual highly mobile aqueous phase of the chassis formulation.

The inventive mixture (I), comprising at least four surfactants (I1) to (I4) within the specified ranges, appears to minimize the presence of any residual highly mobile aqueous phase in the chassis formulation. Consequently, the oxidative hair colouring chassis and/or composition described in the present invention provides effective scalp protection during oxidative hair colouring.

The amount of any residual highly mobile aqueous phase is measured via RFP (Rheology Filter Paper) as described below in the examples.

### DETAILED DESCRIPTION OF THE INVENTION

The following detailed description is merely exemplary in nature and is not intended to limit the presently claimed invention or the application and uses of the presently claimed invention. Furthermore, there is no intention to be bound by any theory presented in the preceding technical field, background, summary or the following detailed description.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

Furthermore, the terms "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the subject matter described herein are capable of operation in other sequences than described or illustrated herein. In case the terms "(A)", "(B)" and "(C)" or "(a)", "(b)", "(c)", "(d)", "(i)", "(ii)" etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

The term "about", when used in relation to a weight ratio or a weight percentage, means that a specific value should be understood as meaning a range of ± 10 %. For example, the weight proportion of a compound of about 1 %, means a weight proportion ranging from 0.9 to 1.1 %. For example, the weight proportion of a compound of about 2.5 %, means a weight proportion ranging from 2.25 to 2.75 %. In another embodiment the term "about" can mean the exact value.

In the following passages, different aspects of the subject matter are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "preferred embodiment" means that a particular feature, structure, or characteristic described in connection with the embodiment is included in at least one embodiment of the presently claimed invention. Thus, appearances of the phrases "in one embodiment" or "in a preferred embodiment" in various places throughout this specification are not necessarily all referring to the same embodiment but may refer to different embodiments of the presently claimed invention. Furthermore, the features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments can be used in any combination.

Furthermore, the ranges defined throughout the specification include the end values as well, i.e. a range of 1 to 10 implies that both 1 and 10 are included in the range. For the avoidance of doubt, the applicant shall be entitled to any equivalents according to applicable law.

For the purposes of the presently claimed invention, the sum of wt.% of all the compounds (components), as described herein, in the respective compositions add up to 100 wt.%.

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests, or discloses any such invention. Further, to the extent that any meaning or definition of a term in this document conflicts with any meaning or definition of the same term in a document incorporated by reference, the meaning or definition assigned to that term in this document shall govern.

The measurement techniques described hereinabove and hereinafter are well known to a person skilled in the art and therefore do not limit the presently claimed invention.

By "oxidative hair colouring composition", it is meant a ready-to-use composition which can change the colour of hair on which it is applied, and which preferably comprises an alkalizing agent, an oxidizing agent, and oxidative dyes (oxidative coupler dye precursor and oxidative primary dye precursor).

By "two-component" oxidative hair colouring composition it is meant an oxidative hair colouring composition which is obtained by mixing shortly before use two compositions: a tint composition and an oxidizing composition. The tint composition comprises the oxidative primary dye precursors, oxidative coupler dye precursors and the alkalizing agent. The oxidizing composition comprises the oxidizing agent. The term "two-component oxidative hair colouring composition" and the term "oxidative hair colouring composition" in the present case are used synonymously.

By "composition" it is meant a composition which is mixed by the user with one or more other composition for preparing the ready-to-use oxidative hair colouring composition to be applied to the hair. Hereinafter, the "hair colouring composition" refers to a use/mixed hair colouring composition.

By "user" it is meant the person preparing the hair colouring composition. The user is for example a professional hair stylist working in a salon and is different from the subject on whose hair the composition is applied, or the user is identical to the person on whose hair the composition is applied. The mixing can take place in a bowl or within a bottle or even through a mixer placed between the stored individual compositions and the point at which it is dispensed to be used. While two component oxidative hair colouring compositions are the most commonly found in the market, there are also others which use three or more components. For example, these colouring compositions comprise a tint composition and a separate alkali composition both of which are mixed in combination with the oxidizing composition to produce the colouring composition. In such a three-component system, the tint composition and the alkali composition are considered to be equivalent to the tint composition of a two-component system.

Alternatively, a tint composition, an anhydrous oxidizer composition and an aqueous composition can be mixed with the anhydrous composition to create the finished mixed colouring composition with the tint composition. In such a three-component system, the anhydrous oxidizer composition and the aqueous composition form the oxidizer composition of the two-component system. An anhydrous dye powder, tablet or granule can also be mixed with an aqueous alkali composition and an oxidizing composition.

Those skilled in the art are aware that there are multiple combinations of the components that can be interchanged to obtain the final mixed oxidative hair colouring composition. All these combinations are within the scope of the presently claimed invention which is exemplified in a non-limiting way.

By "lift" (or "lift power") it is meant the amount of lightening caused by the bleaching of the natural hair pigment melanin by using the oxidative hair colouring composition of the presently claimed invention. The amount of lift provided by different oxidative hair colouring compositions can be compared by using a human natural dark hair sample (e.g., dark hair of an individual of Chinese descent) and measuring the change of colour achieved following application of the compositions. The change in colour can be measured using well known parameters such as L*a*b* values. A composition can be said to provide a higher lift than another composition, when the resulting L* value or the so-called dL* value (given by L*final - L*initial) measured for a given treated sample of dark hair is higher for that composition than for the other composition, using the same experimental conditions. The denomination Level 2 (herein used interchangeably with "demi-permanent" or "tone-on-tone") and Level 3 (herein used interchangeably with "permanent") are commonly used in the hair colour trade to differentiate compositions with medium and high lift.

By "oxidizing agent" it is meant an electron accepting compound suitable for use in hair colouring compositions for removing the natural colour of hair (by destroying the melanin pigment) and reacting with oxidative primary dye precursors to enable the reaction with the oxidative coupler dye precursors to form the colour dye products. The most commonly used oxidizing agent in the art is hydrogen peroxide, however further suitable oxidizing agents that can be used in combination with hydrogen peroxide are described below. Oxidizing agents also include those which produce hydrogen peroxide when contacted with water. For example, sodium percarbonate, sodium perborate and urea peroxide can be used as anhydrous materials, which when contacted with water release hydrogen peroxide. For such oxidizing agents which product hydrogen peroxide when contacted with water, the wt.% of hydrogen peroxide within mixed colouring composition is considered as the level of oxidizing agent (E).

By "alkalizing agent" it is meant one or more compounds suitable for raising the pH to alkaline level in mixed colouring compositions, in particular to a pH in the range of ≥ 8.5 to ≤ 10.5. Generally, the most commonly used alkalizing agents in the art are ammonia and monoethanolamine, however in the presently claimed invention also alkalizing agent other than ammonia (herein "non-ammonia, non-monoethanolamine" alkalizing agent), can be used. The alkali agent is preferably used to raise the pH of the mixed colouring composition to a pH in the range of ≥ 8.5 to ≤ 10.5.

In a preferred embodiment the oxidative hair colouring composition of the presently claimed invention does not contain ammonia.

The below materials are being used in the embodiments of the presently claimed invention. The presently claimed invention is described with non-limiting embodiments. The listed ingredients are the preferred features as well as other preferred but non-limiting features of the presently claimed invention.

### Mixture (I)

In one embodiment, the presently claimed invention is directed to a mixture (I) comprising at least four surfactants (I1) to (I4), wherein the mixture (I) comprises
(I1) 5 to 15 wt.% of at least one first non-ionic surfactant having an HLB value in the range of 1 to 9,
(I2) 45 to 65 wt.% of at least one second non-ionic surfactant having an HLB value in the range of higher than 9 to 14,
(I3) 10 to 30 wt.% of at least one third non-ionic surfactant having an HLB value in the range of higher than 14 to 20,
(I4) 10 to 30 wt.% of at least one anionic surfactant having an HLB value of higher than 17,
wherein the % by weight values in each case are based on the total weight of the surfactants (I1), (I2), (I3) and (I4).

The HLB (Hydrophilic-Lipophilic Balance) value represents a surfactant's affinity for water or oil. In this context, it refers to the HLB value at 25 °C, as defined by Griffin. The Griffin HLB system is well known to those skilled in the art and is described in J. Soc. Cosmet. Chem., 1954, 5:249-256. Non-ionic surfactants typically have HLB values ranging from 0 to 20, with values near 0 indicating greater lipophilicity and values near 20 indicating greater hydrophilicity. Anionic and cationic surfactants, on the other hand, may exhibit HLB values exceeding 20.

In the present case the terms "(I1)", "component (I1)" and "at least one first non-ionic surfactant" are used synonymously, and therefore have preferably the same meaning. In the present case, also the terms "(I2)", "component (I2)" and "at least one second non-ionic surfactant" are used synonymously, and therefore have preferably the same meaning. Also, the terms "(I3)", "component (I3)" and "at least one third non-ionic surfactant" are used synonymously, and therefore have preferably the same meaning. The terms "(I4)", "component (I4)" and "at least one anionic surfactant" are used synonymously, and therefore have preferably the same meaning.

The term "at least one surfactant" means exactly one surfactant and, also a mixture of two or more different surfactants. The term "at least four surfactants" means exactly four different surfactants and, also a mixture of four or more different surfactants.

In a more preferred embodiment, the presently claimed invention is directed to a mixture (I) comprising at least four surfactants (I1) to (I4), wherein the mixture (I) comprises
(I1) 7 to 13 wt.% of at least one first non-ionic surfactant having an HLB value in the range of 1 to 9,
(I2) 50 to 60 wt.% of at least one second non-ionic surfactant having an HLB value in the range of higher than 9 to 14,
(I3) 12 to 28 wt.% of at least one third non-ionic surfactant having an HLB value in the range of higher than 14 to 20,
(I4) 12 to 28 wt.% of at least one anionic surfactant having an HLB value of higher than 17,
wherein the % by weight values in each case are based on the total weight of the surfactants (I1), (I2), (I3) and (I4).

In a further preferred embodiment, the weight ratio of the total weight of the non-ionic surfactants to the total weight of the anionic surfactants is in the range from 3:1 to 8:1 [g/g], preferably in the range from 4:1 to 7:1.

### First non-ionic surfactant (I1)

The at least one first non-ionic surfactant (I1) having an HLB value in the range of 1 to 9 can be selected from the group consisting of fatty acid mono- or diesters of glycols, sugar alcohols and glycerol, and esters of beeswax and polyethylene glycol.

### Fatty acids

In the context of the present invention the term "fatty acid" means saturated or unsaturated carboxylic acids having 10 to 30 carbon atoms. Preferably, the saturated fatty acids are selected from the group consisting of lauric acid (C12), myristic acid (C14), palmitic acid (C16), stearic acid (C18), arachidic acid (C20) and behenic acid (C22). Monounsaturated fatty acids are preferably selected from the group consisting of oleic acid (C18:1) and palmitoleic acid (C16:1). Polyunsaturated fatty acids are preferably selected from the group consisting of linoleic acid (C18:2), alpha-linolenic acid (C18:3), arachidonic acid (C20:4), eicosapentaenoic acid (EPA) (C20:5) and docosahexaenoic acid (DHA) (C22:6).

### Glycols

In the context of the present invention the term "glycol" means an aliphatic diol. Examples for aliphatic diols are ethylene glycol (ethane-1,2-diol), propane-1,3-diol and 1,4-butanediol.

### Sugar alcohols

In the context of the present invention the term "sugar alcohol" means organic compounds, typically derived from sugars, containing one hydroxyl group (-OH) attached to each carbon atom. They can occur naturally or be produced industrially by hydrogenating sugars. Examples for sugar alcohols are xylitol and sorbitol.

### Fatty acid mono- or diesters of glycols, sugar alcohols and glycerol

An example of a fatty acid ester of glycol is glycol stearate, an ester of stearic acid and ethylene glycol. An example of a fatty acid ester of a sugar alcohol is sorbitan monostearate, an ester of sorbitol and stearic acid. An example for a fatty acid ester of glycerol is glyceryl monostearate, the glycerol ester of stearic acid.

The at least one first non-ionic surfactant (I1) is more preferably selected from the group consisting of glycol distearate (ethane-1,2-diyl di(octadecanoate)), sorbitan monostearate, glyceryl monostearate, sorbitan monooleate, sorbitan trioleate, beeswax PEG-8 esters, and propylene glycol stearate, most preferably the at least one first non-ionic surfactant (I1) is glycol distearate.

### Second non-ionic surfactant (I2)

The at least one second non-ionic surfactant (I2) having an HLB value in the range of higher than 9 to 14 can be selected from the group consisting of alkanolamines of fatty acids, ethoxylated fatty acid esters of glycerol, condensation products of fatty alcohols and sugar and ethoxylated fatty alcohols.

### Alkanolamines

In the context of the present invention the term "alkanolamine" means organic compounds that contain both hydroxyl (-OH) and amino (-NH₂, -NHR, and -NR₂) functional groups on an alkane backbone. Examples for alkanolamines are methanolamine, monoethanolamine (2-aminoethanol), diethanolamine (2,2'-Azanediyldi(ethan-1-ol)), monoisopropanolamine (1-Aminopropan-2-ol), and aminomethyl propanol (2-Amino-2-methylpropan-1-ol).

### Ethoxylated fatty acid esters

In the context of the present invention the term "ethoxylated fatty acid esters" means organic compounds that are produced by ethoxylation of fatty acid esters. "Ethoxylation" means in this case a chemical reaction in which ethylene oxide (C₂H₄O) adds to a fatty acid ester. An example for an ethoxylated fatty acid ester is PEG-7 glyceryl cocoate, with 7 units of ethylene oxide monomer in the polymer chain. Glyceryl cocoate is formed from glycerol and fatty acid obtained from coconut oil.

### Ethoxylated fatty alcohols

In the context of the present invention the term "ethoxylated fatty alcohols" means organic compounds that are produced by ethoxylation of fatty alcohols. "Ethoxylation" means in this case a chemical reaction in which ethylene oxide (C₂H₄O) adds to a fatty alcohol. An example for an ethoxylated fatty alcohol is laureth-4 with 4 units of ethylene oxide monomer, that is formed by the ethoxylation of lauryl alcohol.

The term "fatty alcohols" in the context of the present invention means straight-chain primary alcohols, having 4 to 26 carbon atoms, derived from natural fats and oils. Examples for fatty alcohols are lauryl alcohol, stearyl alcohol, and oleyl alcohols.

The at least one second non-ionic surfactant (I2) is preferably selected from the group consisting of cocamide monoethanolamine, cocamide diethanolamine, cocamide monoisopropanolamine, lauramide monoethanolamine, PEG-7 glyceryl cocoate, PEG-6 caprylic glycerides, lauryl glucoside, decyl glucoside, cetearyl glucoside, laureth-4, and PEG-8 caprylic glycerides, more preferably the at least one second non-ionic surfactant (I2) are cocamide monoethanolamine and PEG-7 glyceryl cocoate.

### Third non-ionic surfactant (I3)

The at least one third non-ionic surfactant (I3) having an HLB value in the range of higher than 14 to 20 can be selected from the group consisting of fatty alcohols, ethoxylated fatty alcohols, polysorbates and ethoxylated oils.

### (Ethoxylated) fatty alcohols

Here, the same applies as under the at least one second non-ionic surfactant (I2) with the difference that (ethoxylated) fatty alcohols of the at least one second non-ionic surfactant (I2) have an HLB value in the range of higher than 9 to 14, while the (ethoxylated) fatty alcohols of the at least one third non-ionic surfactant (I3) have an HLB value in the range of higher than 14 to 20. An example for a fatty alcohol (I3) is cetearyl alcohol, a mixture of fatty alcohols which consists predominantly of cetyl and stearyl alcohols. An example for an ethoxylated fatty alcohol (I3) is ceteareth-25, a mixture of ethoxylated fatty alcohols which consists predominantely of ethoxylated cetyl alcohol and ethoxylated stearyl alcohol with 25 units of ethylene oxide monomer.

### Polysorbates

The term "polysorbates" in the context of the present invention means compounds derived from ethoxylated sorbitan, esterified with fatty acids. An example for a polysorbate (I3) is polysorbate 80 which derives from polyethoxylated sorbitan and oleic acid.

### Ethoxylated oils

An example for a suitable ethoxylated oil is ethoxylated castor oil.

The at least one third non-ionic surfactant (I3) is preferably selected from the group consisting of cetearyl alcohol, ceteareth-25 (ethoxylated C16-18 alcohols), polysorbate 80, and PEG-40 hydrogenated castor oil, more preferably the at least one third non-ionic surfactant (I3) is ceteareth-25.

### Anionic surfactant (I4)

The at least one anionic surfactant having an HLB value of higher than 17 can be selected from the group consisting of alkyl sulfates, alkyl ether sulfates, alkyl sulphonates, alkaryl sulfonates, α-olefin-sulphonates, alkylamide sulphonates, alkarylpolyether sulphates, alkylamidoether sulfates, alkyl monoglyceryl ether sulfates, alkyl monoglyceride sulfates, alkyl monoglyceride sulfonates, alkyl succinates, alkyl sulfosuccinates, alkyl ether sulfosuccinates, alkyl sulfosuccinamates, alkyl amidosulfosuccinates, alkyl sulfoacetates, alkyl phosphates, alkyl ether phosphates, alkyl ether carboxylates, alkyl amidoethercarboxylates, acyl lactylates, alkyl isethionates, acyl isethionates, carboxylate salts and amino acid derived surfactants such as N-alkyl amino acids, N-acyl amino acids, as well as alkyl peptides.

The cation moiety of the forgoing surfactants can be selected from sodium, potassium, magnesium, ammonium, and alkanolammonium ions such as monoethanolammonium, diethanolammonium triethanolammonium ions, as well as monoisopropylammonium, diisopropylammonium and triisopropylammonium ions. In one embodiment, the alkyl and acyl groups of the foregoing surfactants contain from about 6 to about 24 carbon atoms in one aspect, from 8 to 22 carbon atoms in another aspect and from about 12 to 18 carbon atoms in a further aspect and may be unsaturated. The aryl groups in the surfactants are preferably selected from phenyl or benzyl. The ether containing surfactants set forth above can contain from 1 to 10 ethylene oxide and/or propylene oxide units per surfactant molecule in one aspect, and from 1 to 3 ethylene oxide units per surfactant molecule in another aspect.

Preferably, the at least one anionic surfactant having an HLB value of higher than 17 are selected from the group consisting of sodium, potassium, lithium, magnesium, and ammonium salts of laureth sulfate, trideceth sulfate, myreth sulfate, C12-C13 pareth sulfate, C12-C14 pareth sulfate, and C12-C15 pareth sulfate, ethoxylated with 1, 2, and 3 moles of ethylene oxide; the sodium potassium, lithium, magnesium, ammonium, and triethanolammonium salts of lauryl sulfate, coco sulfate, tridecyl sulfate, myristyl sulfate, cetyl sulfate, cetearyl sulfate, stearyl sulfate, oleyl sulfate, and tallow sulfate, disodium lauryl sulfosuccinate, disodium laureth sulfosuccinate, sodium cocoyl isethionate, sodium lauroyl isethionate, sodium lauroyl methyl isethionate, sodium C12-C14 olefin sulfonate, sodium laureth-6 carboxylate, sodium dodecylbenzene sulfonate, triethanolamine monolauryl phosphate, and fatty acid soaps, including the sodium, potassium, ammonium, and triethanolamine salts of a saturated and unsaturated fatty acids containing from about 8 to about 22 carbon atoms such as sodium stearate.

The at least one anionic surfactant (I4) is more preferably selected from the group consisting of sodium laureth sulfate (α-sulfo-ω-(dodecyloxy)-poly(oxyethane-1,2-diyl), sodium salt), sodium lauryl sulfate, ammonium lauryl sulfate, sodium stearate, sodium myreth sulfate and sodium coco-sulfate, most preferably the at least one anionic surfactant (I4) is sodium laureth sulfate.

The respective HLB values of the surfactants (I1), (I2), (I3) and (I4) are shown in the following table 1.

| **First non-ionic surfactant (I1)** | **HLB value** |
|---|---|
| glycol distearate | 1 |
| sorbitan monostearate | 4.7 |
| glyceryl monostearate | 3.8 |
| sorbitan monooleate | 4.3 |
| sorbitan trioleate | 1.8 |
| beeswax PEG-8 esters | 9 |
| propylene glycol stearate | 3 to 4 |

| **Second non-ionic surfactant (I2)** | |
|---|---|
| cocamide monoethanolamine | 13.5 |
| cocamide diethanolamine | 13.5 |
| PEG-7 glyceryl cocoate | 11 |
| PEG-6 caprylic glycerides | 12.5 to 14 |
| lauryl glucoside | 10 to 12 |
| decyl glucoside | 12.1 |
| cetearyl glucoside | 11 |
| laureth-4 | 9.7 |
| PEG-8 caprylic glycerides | 12 |

| **Third non-ionic surfactant (I3)** | |
|---|---|
| cetearyl alcohol | 15.5 |
| ceteareth-25 | 16 |
| polysorbate 80 | 15 |
| PEG-40 hydrogenated castor oil | 15 |

| **Anionic surfactant (I4)** | |
|---|---|
| Laureth sulfate | 40 |
| Sodium lauryl sulfate | 40 |
| Ammonium lauryl sulfate | 31 |
| Sodium stearate | 18 |
| Sodium myreth sulfate | 38 |

The HLB values provided for the listed surfactants are based on widely recognized standard references in the cosmetics and surfactant industry. However, it is important to note that the HLB values of certain surfactants may slightly vary depending on the specific source, manufacturing process, or grade of the material. Therefore, for certain surfactants, a range is given to account for these variations while ensuring the values remain reliable and applicable for formulation purposes.

The mixture (I) can be used for protecting the scalp during oxidative hair colouring and/or for providing high lightening during oxidative hair colouring.

### Oxidative hair colouring chassis

In one embodiment the presently claimed invention is directed to an oxidative hair colouring chassis comprising the mixture (I) and at least one of the following components:
(F) at least one cosmetically acceptable aqueous or aqueous-organic medium and
(H) at least one fatty substance.

Preferably, the oxidative hair colouring chassis does not comprise any oxidative coupler dye precursors (component (A)), any oxidative primary dye precursors (component (B)) or any oxidizing agents (component (E)).

The mixture (I) is preferably present in an amount in the range of ≥ 0.1 wt.% to ≤ 30 wt.%, more preferably in an amount in the range of ≥ 15 wt.% to ≤ 25 wt.%, based on the total weight of the oxidative hair colouring chassis.

### Component (F)

In the present case the terms "(F)", "component (F)" and "at least one cosmetically acceptable aqueous or aqueous-organic medium" are used synonymously, and therefore have preferably the same meaning. The term "at least one cosmetically acceptable aqueous or aqueous-organic medium" means exactly one cosmetically acceptable aqueous or aqueous-organic medium and, also a mixture of two or more different cosmetically acceptable aqueous or aqueous-organic mediums.

In a preferred embodiment, the oxidative hair colouring chassis comprises the cosmetically acceptable aqueous or aqueous-organic medium (component (F)) in an amount in the range of ≥ 30.0 to ≤ 90.0 wt.%, preferably in an amount in the range of ≥ 35.0 to ≤ 85.0 wt.%, more preferably in an amount in the range of ≥ 40.0 to ≤ 80.0 wt.%, based on the total weight of the oxidative hair colouring composition.

If component (F) is a cosmetically acceptable aqueous medium it preferably consists of water. The cosmetically acceptable aqueous-organic medium (component (F)) preferably consists of water and a water miscible organic solvent such as an alcohol. The cosmetically acceptable aqueous-organic medium (component (F)) preferably comprises water and a monoalcohol such as ethanol and isopropanol and/or a polyalcohol such as propylene glycol, hexylene glycol, glycerin, and propane diol.

In a preferred embodiment component (F) consists of water.

### Component (H)

In the present case the terms "(H)", "component (H)" and "at least one fatty substance" are used synonymously, and therefore have preferably the same meaning. The term "at least one fatty substance" means exactly one fatty substance and, also a mixture of two or more different fatty substances.

In a preferred embodiment of the presently claimed invention, the fatty substances are selected from the group consisting of liquid hydrocarbons, non-silicone oils of animal, plant, mineral or synthetic origin, fatty alcohols, fatty acid esters and/or fatty alcohol esters, non-silicone waxes, and silicones or mixtures thereof, more preferably component (H) is selected from linear or branched C14 to C30 fatty alcohols, most preferably selected from the group consisting of cetearyl alcohol, cetyl alcohol, stearyl alcohol, cetostearyl alcohol or behenyl alcohols or mixtures thereof, especially preferably cetearyl alcohol.

Preferably, the oxidative hair colouring chassis comprises from ≥ 0.1 wt.% to ≤ 20 wt.%, more preferably from ≥ 0.1 wt.% to ≤ 15 wt.%, of component (H).

Therefore, in a preferred embodiment, mixture (I) is present in an amount in the range of ≥ 0.1 wt.% to ≤ 30 wt.%, component (F) is present in an amount in the range of ≥ 30 wt.% to ≤ 90 wt.%, and component (H) is present in an amount in the range of ≥ 0.1 wt.% to ≤ 20 wt.%, each based on the total weight of the oxidative hair colouring chassis.

In a preferred embodiment, the oxidative hair colouring chassis further comprises the following components
(C) at least one chelant and
(D) at least one alkalizing agent.

### Component (C)

In the present case the terms "(C)", "component (C)", "at least one chelant (C)", and "at least one chelant" are used synonymously, and therefore have preferably the same meaning. The term "at least one chelant" means exactly one chelant and, also mixtures of two or more chelants.

The at least one chelant preferably comprises diethylenetriamine penta(methylene phosphonic acid), and salts thereof.

Diethylenetriamine penta(methylene phosphonic acid) is also known under the IUPAC name as {[(Phosphonomethyl)azanediyl]bis[ethane-2,1-diylnitrilobis(methylene)]}tetrakis (phosphonic acid) and has the CAS number 15827-60-8. The abbreviation for diethylenetriamine penta(methylene phosphonic acid) is DTPMP.

DTPMP is normally used as salts, because the acid form has very limited solubility in water and tends to crystallize in concentrated aqueous solutions. Preferably the pentasodium salt of DTPMP is used having the CAS number 22042-96-2.

The term "diethylenetriamine penta(methylene phosphonic acid) and ethylenediamine-N,N'-disuccinic acid" and "DTPMP" in the present invention includes the free acid as well as salts of DTPMP.

Component (C) of the inventive oxidative hair colouring chassis, in addition to DTPMP, in one embodiment preferably contains one, two or more additional chelants selected from the group consisting of ethylenediamine-N,N'-disuccinic acid (EDDS), ethylenediaminetetraacetic acid (EDTA), hydroxyethyl ethylenediamine triacetic acid (HEDTA), ethylenediamine-N,N'-diglutaric acid (EDDG), 2-hydroxypropylenediamine-N,N'-disuccinic acid (HPDDS), N,N-dicarboxymethylglutamic acid (GLDA), glycinamide-N,N'-disuccinic acid (GADS), ethylenediamine-N-N'-bis(ortho-hydroxyphenyl acetic acid) (EDDHA), dimethyl glucamine (DMG), N-(1-carboxyethyl)iminodiacetic acid, methylglycine N,N-diacetic acid (MGDA), amino trimethylene phosphonic acid (ATMP) and their salts thereof.

In a preferred embodiment component (C) comprises DTPMP, EDDS and EDTA, and salts thereof.

Ethylenediamine-N,N'-disuccinic acid has the CAS number 20846-91-7. The abbreviation for ethylenediamine-N,N'-disuccinic acid is EDDS. EDDS has two chiral centers, and as such three stereoisomers. These are the enantiomeric (R,R) and (S,S) isomers and the achiral meso (R,S) isomer.

The term "ethylenediamine-N,N'-disuccinic acid" and "EDDS" in the present invention includes the free acid as well as salts of EDDS. Preferably in the presently claimed invention (S,S) ethylenediamine-N,N'-disuccinic acid ((S,S) EDDS) is used.

The term "ethylenediamine-N,N'-disuccinic acid" and "EDDS" in the present invention includes the free acid as well as salts of EDDS.

EDTA is the abbreviation for ethylenediaminetetraacetic acid having the CAS number 60-00-4. EDTA is available as free acid or in the form of several salts, like disodium EDTA, sodium calcium edetate, and tetrasodium EDTA.

The term "ethylenediaminetetraacetic acid" and "EDTA" in the present invention includes the free acid as well as salts of EDTA.

All weight amounts (wt.%) given in the present invention in view of chelants comprised in component (C) are in each case calculated on basis of the chelants in form of the free acid.

In other words, if the amount of DTPMP comprised in the oxidative colouring chassis in wt.% is given, the amount of DTPMP in wt.% is calculated based on DTMP in form of a free acid and not based on DTPMP in form of a salt. The same holds true for EDDS, EDTA and any other chelant that might be comprised in the oxidative colouring chassis.

In a preferred embodiment the oxidative hair colouring chassis comprises the at least one chelant (component (C)) in an amount in the range from ≥ 0.20 wt.% to ≤ 4 wt.%, based on the total weight of the oxidative hair colouring chassis.

The amount of component (C) at present means the amount of all chelants comprised in component (C), preferably comprised in the oxidative hair colouring chassis.

In an especially preferred embodiment component (C) comprises a mixture of
0.1 to 3 wt.% of DTPMP,
0.1 to 0.75 wt.% of EDDS, and
0.01 to 0.25 wt.% of EDTA,
wherein the wt.% values are based on the total weight of the oxidative hair colouring chassis.

In a particularly preferred embodiment component (C) comprises a mixture of
0.3 to 2.5 wt.% of DTPMP,
0.2 to 0.75 wt.% of EDDS, and
0.05 to 0.25 wt.% of EDTA,
wherein the wt.% values are based on the total weight of the oxidative hair colouring chassis.

In a preferred embodiment the chelant mixture (component (C)) beside DTPMP, EDDS and optionally EDTA contains less than 10 wt.-% of further chelant, based on the total weight of component (C) comprised in the oxidative hair colouring chassis.

In a more preferred embodiment the chelant mixture (component (C)) beside DTPMP, EDDS and optionally EDTA does not contain a further chelant. In another preferred embodiment the oxidative hair colouring chassis beside DTPMP, EDDS and optionally EDTA does not contain a further chelant.

### Component (D)

In the present case the terms "(D)", "component (D)" and "at least one alkalizing agent" are used synonymously, and therefore have preferably the same meaning. The term "at least one alkalizing agent" means exactly one alkalizing agent and, also a mixture of two or more different alkalizing agents.

In a preferred embodiment in the oxidative hair colouring chassis component (D) is at least one alkalizing agent selected form the group consisting of 2-amino-1-propanol, 2-amino-2-methylpropan-1-ol and monoethanolamine, wherein monoethanolamine is especially preferred.

In a preferred embodiment component (D) does not contain ammonia. In another preferred embodiment the oxidative hair colouring chassis does not contain ammonia.

All weight amounts (wt.%) given in the present invention in view of the at least on alkalizing agent (component (D)) are in each case calculated on basis of the alkalizing agent in form of the free base.

In one embodiment component (D) is present in an amount in the range of ≥ 0.1 wt.% to ≤ 20.0 wt.%, preferably in an amount in the range of ≥ 0.1 wt.% to ≤ 15.0 wt.%, in each case based on the total weight of the oxidative hair colouring chassis.

In a preferred embodiment the component (D) beside monoethanolamine, contains less than 10 wt.-% of further alkalizing agent(s) based on the total weight of component (D) comprised in the oxidative hair colouring chassis.

Optionally, the oxidative hair colouring chassis can also comprise
(J) at least one thickener.

### Component (J)

In the present case the terms "(J)", "component (J)" and "at least one thickener" are used synonymously, and therefore have preferably the same meaning. The term "at least one thickener" means exactly one thickener and, also a mixture of two or more different thickeners.

The oxidative hair colouring chassis of the presently claimed invention preferably comprises a thickener, in particular a polymeric thickener in an amount that is sufficient to impart a viscosity to the composition that allows for its ready application to hair without unduly dripping off the hair, as is known in the art. Typically, such an amount will be at least about 0.1 wt.%, in some embodiments, at least about 0.5 wt.%, in other embodiments, at least about 1.0 wt.%, based on the total weight of the oxidative hair colouring chassis.

Examples of commonly used associative polymeric thickeners are sold under the tradename Aculyn-22 and Aculyn-33 by the company Rohm & Haas, Permulen TR1, Carbopol 2020, Carbopol Ultrez-21 by the company Noveon, and Structure 2001 and Structure 3001 by the company National Starch. Other suitable polymers include polyether polyurethanes, for example Aculyn-44 and Aculyn-46 by the company Rohm and Haas. Another suitable associative polymer is cellulose modified with groups comprising at least one C8 - C30 fatty chain, such as the product Natrosol Plus Grade 330 CS sold by the company Aqualon.

In one embodiment of the presently claimed invention the hair colouring chassis comprises salt tolerant thickeners, including but not limited to: xanthan, guar, hydroxypropyl guar, scleroglucan, methyl cellulose, ethyl cellulose (available as AQUACOTE^{®}, hydroxyethyl cellulose (NATROSOL^{®}), carboxymethyl cellulose, hydroxypropylmethyl cellulose, microcrystalline cellulose, hydroxybutylmethyl cellulose, hydroxypropyl cellulose (available as KLUCEL^{®}), hydroxyethyl ethyl cellulose, cetyl hydroxyethyl cellulose (available as NATROSOL^{®} Plus 330), N-vinylpyrrolidone (available as POVIDONE^{®}), Acrylates/Ceteth-20 Itaconate Copolymer (available as STRUCTURE^{®} 3001), hydroxypropyl starch phosphate (available as STRUCTURE^{®} ZEA), polyethoxylated urethanes or polycarbamyl polyglycol ester (e.g. PEG-150/Decyl/SMDI copolymer (e.g. ACULYN^{®} 44), PEG- 150/Stearyl/SMDI copolymer (available as ACULYN^{®} 46), trihydroxystearin (available as THIXCIN^{®}), acrylates copolymer (e.g. available as ACULYN^{®} 33) or hydrophobically modified acrylate copolymers (e.g. Acrylates / Steareth-20 Methacrylate Copolymer (available as ACULYN^{®} 22), acrylates/steareth-20 methacrylate crosspolymer (available as ACULYN^{®} 88), acrylates/vinyl neodecanoate crosspolymer (available as ACULYN^{®} 38), acrylates/beheneth-25 methacrylate copolymer (available as ACULYN^{®} 28), acrylates/C 10-30 alkyl acrylate crosspolymer (available as Carbopol^{®} ETD 2020), non-ionic amphophilic polymers comprising at least one fatty chain and at least one hydrophilic unit selected from polyether urethanes comprising at least one fatty chain.

In one embodiment of the presently claimed invention, the oxidative hair colouring chassis comprises at least one mineral thickener selected from organophilic clays, fumed silicas, or mixtures thereof. The at least one mineral thickener is present in an amount ranging from ≥ 1.0 to ≤ 30 wt.%, based on the total weight of the oxidative hair colouring chassis.

In one embodiment of the presently claimed invention, the organic thickener is selected from cellulose-based thickeners (hydroxyethycellulose, hydroxypropyl cellulose, carboxymethylcellulose), guar gum and derivatives thereof (hydroxypropyl guar), gums of microbial origin (xanthan gum, scleroglucan gum), crosslinked homopolymers of acrylic acid or of acrylamidopropanesulfonic acid, and cellulose-based thickeners, such as hydroxyethylcellulose. The at least one organic thickener is present in an amount ranging from ≥ 0.1 to ≤ 20 wt.%, based on the total weight of the oxidative hair colouring chassis.

The oxidative hair colouring chassis preferably has a pH in the range of ≥ 8.5 to ≤ 11.5.

The oxidative hair colouring chassis may for example be formulated and delivered as aqueous hair product, emulsion, gel, aerosol, or foam, preferably as gel.

### Oxidative hair colouring composition

In one embodiment the presently claimed invention is also directed to an oxidative hair colouring composition comprising an inventive oxidative hair colouring chassis and the following components
(A) at least one oxidative coupler dye precursor,
(B) at least one oxidative primary dye precursor, and
(E) at least one oxidizing agent.

Common ingredients for oxidative hair colouring formulations include, but are not limited to: alkalizers, solvents; water, oxidative dyes; direct dyes; oxidizing agents, like hydrogen peroxide; radical scavengers; thickeners and/or rheology modifiers; chelants; pH modifiers and buffering agents; carbonate ion sources; peroxymonocarbonate ion sources; anionic, cationic, non-ionic, amphoteric or zwitterionic surfactants, or mixtures thereof; anionic, cationic, non-ionic, amphoteric or zwitterionic polymers, or mixtures thereof; fragrances; enzymes; dispersing agents; peroxide stabilizing agents; antioxidants; catalysts, natural ingredients, e.g. proteins and protein derivatives, and plant extracts; conditioning agents including silicones and cationic polymers, ceramides, preserving agents, pigments, and opacifiers and pearling agents (such as titanium dioxide and mica). Some adjuvants referred to above, but not specifically described below, which are suitable are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions) are useful in identifying specific adjuvants to achieve a particular purpose or multipurpose.

The oxidative hair colouring compositions of the presently claimed invention preferably further comprise any usual hair colouring chassis ingredients and may use any common ingredients, known in the field as long as they are compatible with the requirements set in the claims.

### Component (A)

In the present case the terms "(A)", "component (A)", and "at least one oxidative coupler dye precursor (A)", are used synonymously, and therefore have preferably the same meaning. The term "at least one oxidative coupler dye precursor" means exactly one oxidative coupler dye precursor and, also mixtures of two or more oxidative coupler dye precursors.

In one embodiment of the presently claimed invention, the oxidative hair colouring composition comprises as component (A) at least one oxidative coupler dye precursor selected from the group consisting of resorcinol, 4-chlororesorcinol, 2-chlororesorcinol, 2-methylresorcinol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, hydroxybenzomorpholine, 2-amino-5-ethylphenol, 6-amino-m-cresol, 6-amino-o-cresol, 2,4-diaminophenoxyethanol, 2-amino-4-hydroxyethylaminoanisole, 1,3-bis-(2,4-diaminophenoxy)propane, 2,6-dihydroxyethylaminotolueneµ, m-phenylenediamine, 2,4-diamino-1,5-di(2-hydroxyethoxybenzene, 1-naphthol, 2-methyl-1-naphthol, 1,5-naphthalenediol, 2,7-naphthalenediol, 1-acetoxy-2-methyl naphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindole, 6-hydroxyindole, dihydroxyindoline, phenyl methyl pyrazolone, 1,2,4-trihydroxybenzene, 5-((2-hydroxyethyl)amino)-1,3-benzodioxol, isatin, hydroquinone, 4-formyl-1-methylquinolinium-p-toluenesulfonate and salts thereof.

These and other primary dye precursors and coupler dye precursors (component (A)) may be used in different combination to achieve the nuance sought, as is known in the art.

In a preferred embodiment of the presently claimed invention, the oxidative hair colouring composition comprises as component (A) at least one oxidative coupler dye precursor selected from the group consisting of resorcinol, 4-chlororesorcinol, 2-chlororesorcinol, 2-methylresorcinol, m-aminophenol, hydroxybenzomorpholine, 2,4-diaminophenoxyethanol, 2-methyl-5-hydroxyethylaminophenol, 5-((2-hydroxyethyl)amino)-1,3-benzodioxol and salts thereof.

All weight amounts (wt.%) given in the present invention in view of oxidative coupler dye precursors comprised in component (A) are in each case calculated on basis of the oxidative coupler dye precursors in form of the free base.

In the tint composition component (A) is generally present in an amount of 0.001 to 3 % by weight based on the total weight of the tint composition.

In the oxidative hair colouring composition component (A) is generally present in an amount of 0.001 to 1.5 % by weight, preferably in an amount of 0.001 to 1.1 % by weight and more preferably in an amount of 0.001 to 1.0 % by weight, based on the total weight of the oxidative hair colouring composition.

### Component (B)

In the present case the terms "(B)", "component (B)", and "at least one one oxidative primary dye precursor (B)", are used synonymously, and therefore have preferably the same meaning. The term "at least one oxidative primary dye precursor" means exactly one oxidative primary dye precursor and, also mixtures of two or more oxidative primary dye precursors.

In one embodiment of the presently claimed invention, the oxidative hair colouring composition comprises as component (B) at least one oxidative primary dye precursor selected from the group consisting of toluene-2,5-diamine, p-phenylenediamine, n-phenyl-p-phenylenediamine, N,N-bis(2-hydroxyethyl)-p-phenylenediamine, hydroxyethyl-p-phenylenediamine sulphate, hydroxypropyl bis(n-hydroxyethyl-p-phenylenediamine), 2-methoxymethyl-p-phenylenediamine, 2-chloro-p-phenylenediamine, p-aminophenol, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diaminodihydroxypyrazolopyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and salts thereof.

In a preferred embodiment of the presently claimed invention, the oxidative hair colouring composition comprises as component (B) at least one oxidative primary dye precursor selected from the group consisting of toluene-2,5-diamine, hydroxypropyl-p-phenylenediamine, 2-methoxymethyl-p-phenylenediamine and salts thereof.

All weight amounts (wt.%) given in the present invention in view of oxidative primary dye precursors comprised in component (B) are in each case calculated on basis of the oxidative primary dye precursors in form of the free base.

In the tint composition component (B) is generally present in an amount of 0.001 to 7.5 % by weight based on the total weight of the tint composition.

In the oxidative hair colouring composition component (B) is generally present in an amount of 0.001 to 4.0 % by weight, preferably in an amount of 0.001 to 3.0 % by weight and more preferably in an amount of 0.001 to 2.5 % by weight, based on the total weight of the oxidative hair colouring composition.

### Component (E)

In the present case the terms "(E)", "component (E)" and "at least one oxidizing agent" are used synonymously, and therefore have preferably the same meaning. The term "at least one oxidizing agent" means exactly one oxidizing agent and, also a mixture of two or more different oxidizing agents.

The oxidizing agents are preferably water-soluble inorganic peroxide materials which are capable of yielding hydrogen peroxide in an aqueous solution.

In one embodiment of the presently claimed invention, the at least one oxidizing agent is selected from the group consisting of hydrogen peroxide, inorganic alkali metal peroxides (such as sodium periodate and sodium peroxide), organic peroxides (such as urea peroxide, melamine peroxide), inorganic perhydrate salt bleaching compounds (such as the alkali metal salts of perborates, percarbonates, perphosphates, persilicates, persulphates), and mixtures thereof; preferably from the group consisting of hydrogen peroxide, persulphates, and mixtures thereof; most preferably the oxidizing agent is hydrogen peroxide.

"Water-soluble," as defined herein, means that in standard condition at least 0.1 g, 1 g, or 10 g of the oxidizing agent can be dissolved in 1 liter of deionized water.

In one embodiment, the component (E) is preferably present in an amount in the range of ≥ 1.0 wt.% to ≤ 8.0 wt.%, more preferably in an amount in the range from of ≥ 1.0 wt.% to ≤ 6.0 wt.%, more preferably in an amount in the range from of ≥ 1.2 wt.% to ≤ 5.0 wt.%, based on the total weight of the oxidative hair colouring composition.

The oxidizing agents can be provided in aqueous solution or as a powder which is dissolved prior to use.

Optionally, the oxidative hair colouring composition can also comprise
(G) at least one direct dye.

### Component (G)

In the present case the terms "(G)", "component (G)" and "at least one direct dye" are used synonymously, and therefore have preferably the same meaning. The term "at least one direct dye" means exactly one direct dye and, also a mixture of two or more different direct dyes.

Direct dyes may also be incorporated in any of components of the presently claimed invention, in particular in the tint composition. The compositions of the presently claimed invention may also comprise compatible direct dyes, in an amount that is sufficient to provide additional colouring, particularly with regard to intensity. Typically, such an amount will range from 0.005% wt.% to 4.0 wt.%, based on the total weight of the oxidative hair colouring composition. When the oxidative hair colouring composition is obtained by mixing a tint composition and an oxidizing composition, the direct dyes are usually incorporated in the tint composition.

In one embodiment of the presently claimed invention, direct dyes are selected from the group consisting of Acid Yellow 1, Disperse Red 17, Acid Yellow 9, Pigment Red 49, Acid Yellow 11, Acid Black 1, 4-Nitro-1,2-phenylenediamine, Picramic acid, HC Red 13, N,N'-Bis(2-hydroxyethyl)-2-nitro-p-phenylenediamine, HC Yellow 5, HC Red 7, HC Blue 2, HC Yellow 4, HC Yellow 3, HC Blue 1, HC Yellow 2, HC Orange 1, 2-Hydroxyethylamino-5-nitroanisole, 3-Nitro-p-hydroxyethylaminophenol, 3-Methylamino-4-nitrophenoxyethanol, 2-Nitro-5-glyceryl methylaniline, HC Violet 1, HC Orange 2, HC Orange 3, HC Yellow 9, 4-Nitrophenyl aminoethylurea, HC Red 10, HC Red 11, 2-Hydroxyethyl picramic acid, HC Blue 12, HC Yellow 6, Hydroxyethyl-2-nitro-p-toluidine, HC Yellow 12, HC Blue 11, HC Blue 10, HC Blue 6, HC Yellow 7, HC Yellow 10, HC Blue 9, HC Blue 13, 2-Chloro-6-(ethylamino)-4-nitrophenol, 6-Nitro-2,5-pyridinediamine, HC Violet 2, 2-Amino-6-Chloro-4-Nitrophenol, 4-Hydroxypropylamino-3-nitrophenol, Acid Blue 7, HC Yellow 13, HC Red 14, HC Yellow 15, HC Yellow 14, 2,6-Diamino-3-((pyridin-3-yl)azo)pyridine, Basic Orange 69, HC Red 16, Basic violet 2, Basic Red 51, Basic Yellow 87, Basic Orange 31, HC Blue 16, HC red 17, HC Yellow 17, HC blue 18, HC Yellow 16, HC Red 18, HC Orange 6, Basic Orange 1, Basic Red 76, Basic Brown 16, Basic Yellow 57, CI14700, Acid Red 14, Acid Orange 7, Acid Red 88, FD&C Yellow 6, Acid Red 27, Acid Orange 10, Acid Red 33, Acid red 155, Acid Yellow 23, Food Black 2, CI 28440, HC Brown 2, Acid Blue 1, Acid Blue 3, Acid Blue 9, Acid Violet 49, CI 42735, Basic Blue 26, Acid violet 9, Acid Red 92, Acid Yellow 3, Beta-Carotene, CI 50420, Basic Blue 99, 1-Amino-4-hydroxy-9,10-anthracendion, CI 60725, Acid violet 43, Disperse Violet 1, Acid blue 62, Copper phthalocyanine, Carbon black, Disperse Black 9, Lycopene, Hydroxyanthraquinoneaminopropyl methyl morpholinium methosulfate, HC Blue 8, HC Green 1, Curry red, 2-Hydroxy-1,4-naphthoquinone, CI 77289, Solvent Green 7, Lawsonia Inermis Cera, CI 73000, Indigofera tinctoria, Carmines, HC Blue 14, Acid Red 18, CI 42053, Acid Red 52, Acid Green 25, Disperse Blue 377, Pigment Red 57, HC Blue 15, Tetrabromphenol Blue, Cationic Blue 347, Bromthymol blue sodium, Anthocyanins, Chlorophyll in-Copper complex, Annatto, Natural Green 3, Betanin, Capsanthin, Basic Yellow 29 and combinations thereof.

Preferably, the oxidative hair colouring composition of the invention is obtained by mixing a tint composition (i) and an oxidizing composition (ii).

The tint composition (i) comprises preferably the at least one oxidative coupler dye precursor (component (A)), the at least one oxidative primary dye precursor (component (B)), the at least one chelant (component (C)), the at least one alkalizing agent (component (D)) and, the mixture (I), comprising at least four surfactants (I1) to (I4).

The oxidizing composition (ii) preferably comprises the hydrogen peroxide (component (E)).

Such two component systems are commonly used in the art and each component is formulated so that the resulting mixture is an oxidative hair colouring composition according to the invention.

Therefore, the present invention also relates to a kit comprising
(i) a tint composition comprising components (A), (B), (C), (D) and a mixture (I) as defined in any one of the preceding claims, and
(ii) an oxidative composition comprising component (E) as defined any in any one of the preceding claims.

### Packaging

In one embodiment of the presently claimed invention, before use, the tint and the oxidative composition used in the invention are normally packaged separately from one another. The compositions may be packaged in separate primary packages such as plastic bottle, sachet or tube. The components, particularly each component of a two-component composition, may however be packaged separately but within a common secondary package such as a carton or in different compartment of an aerosol or foam bottle, as is known in the trade. A conditioning composition, which can be applied after rinsing of the oxidative hair colouring composition, may also be packaged in such secondary package.

### Method of Hair Dying

Application of the oxidative hair colouring composition to the hair may be undertaken in several ways. Application of the oxidative hair colouring composition may take place on the whole head of hair of an end user. As used herein, the "whole head of hair" means that the hair all over the head from the root of the hair to the tip of the hair is included in the application process. By contrast, the application of the oxidative hair colouring composition may take place only on the root portion of the hair. The application to the root portion of the hair may still be over the entire head of the end user, but application of the oxidative hair colouring composition is applied only to the section of hair closest to the head (root portion), which is between about 0.01 mm to about 40 mm from the scalp of the head. After application to the root portion, product may be applied to the rest of the hair at a later stage to prevent over processing of the hair in the lengths and ends. Also, application may take place on a portion of hair. Application of a portion of hair is commonly referred to as highlighting or lowlighting. The portion of hair may be physically separated from the whole head of hair in a hair bundle or may be a smaller portion of hair than the whole head of hair. A hair bundle may be physically separated from a whole head of hair by a device including a plastic cap through which hair bundles are formed when hair is pulled through orifices in the plastic cap, metal foils encompassing a hair bundle, strand separators applied to hair at the root portion, or similar devices.

When present, an optional conditioning agent can be provided in an additional container. In the latter case, the conditioner can be mixed immediately before use and applied together with the other components, or the content of the additional container can be applied (after an optional rinse step) as a posttreatment immediately after the oxidative hair colouring composition.

According to one method for oxidatively colouring hair, the method comprises mixing a tint composition and an oxidizing composition and optionally a third component comprising a second non-ammonia alkalizing agent together to form a oxidative hair colouring composition, applying the oxidative hair colouring composition to the hair to form a treated hair surface, waiting for a period of 5-50 minutes, such as 20-35 minutes, and then removing the hair colouring composition from the treated hair surface through rinsing with water.

The methods of colouring hair also may further comprise working the oxidative hair colouring composition into the treated hair surface by hand or by a tool for a few minutes to ensure uniform application to the entire treated hair surface. The oxidative hair colouring composition remains on the treated hair surface while the end hair colour develops for a time period of 5 to 50 minutes to form oxidatively coloured hair. The consumer then rinses his/her oxidatively coloured hair thoroughly with tap water and allows it to dry and/or styles the oxidatively coloured hair.

In one embodiment of the presently claimed invention, a method of treating hair with the oxidative hair colouring composition preferably comprises the steps of:
a. providing a tint composition (i) as described herein;
b. providing an oxidizing composition (ii) as described herein;
c. mixing the oxidizing composition (ii) and the tint composition (i) to obtain a mixed oxidative hair colouring composition as described herein;
d. applying the mixed oxidative hair colouring composition for the oxidative dyeing of keratin fibres onto the hair;
e. leaving the composition on the hair for 5 to 50 minutes; and
f. subsequently rinsing the oxidative hair colouring composition from the hair.

In one embodiment of the presently claimed invention, the oxidative hair colouring composition may be obtained by mixing immediately prior to use a tint composition and the oxidizing composition. A sufficient amount of the mixed oxidative hair colouring composition is applied to the hair, according to the hair abundance, generally from 20 to 250 grams depending on the amount of hair to be coloured. Upon such preparation, the oxidative hair colouring composition is applied to the hair to be dyed and remains in contact with the hair for an amount of time effective to dye the hair. Typically, the oxidative hair colouring composition is allowed to act on the hair from 5 to 50, preferably 10 to 40, preferably 20 to 35 minutes, at a temperature ranging from 15 to 50 °C. Thereafter, the hair is rinsed with water to remove the oxidative hair colouring composition and dried. If necessary, the hair is washed with a shampoo and rinsed, e.g., with water or a weakly acidic solution, such as a citric acid or tartaric acid solution, and dried. Optionally, a separate conditioning product may also be provided.

The oxidative hair colouring composition can be applied on hair via an applicator bottle or brush. It can be used on full head or partly on single strands (highlight application) as common highlight applicator foils, caps and special applicators can be used, but also freehand techniques such as balayage, with brush and/or combs can be possible. The oxidative hair colouring composition can also be applied as a mousse via a manual spray, a pressurized container or an aerosol mousse. The oxidative hair colouring composition may be dispensed as a solid form to which water is added to generate the oxidant and form a thickened vehicle suitable for hair colouring.

The invention is associated with one or more of the following advantages:
- High lightening during oxidative hair colouring
- Protection of the scalp during oxidative hair colouring
- a pleasant odour profile while application to the hair

In the following, there is provided a list of embodiments to further illustrate the present disclosure without intending to limit the disclosure to the specific embodiments listed below.

### EMBODIMENTS

1. A mixture (I) comprising at least four surfactants (I1) to (I4), wherein the mixture (I) comprises
   (I1) 5 to 15 wt.% of at least one first non-ionic surfactant having an HLB value in the range of 1 to 9,
   (I2) 45 to 65 wt.% of at least one second non-ionic surfactant having an HLB value in the range of higher than 9 to 14,
   (I3) 10 to 30 wt.% of at least one third non-ionic surfactant having an HLB value in the range of higher than 14 to 20,
   (I4) 10 to 30 wt.% of at least one anionic surfactant having an HLB value of higher than 17,
   wherein the % by weight values in each case are based on the total weight of the surfactants (I1), (I2), (I3) and (I4).
2. The mixture (I) according to embodiment 1, wherein
   the at least one first non-ionic surfactant (I1) is selected from the group consisting of glycol distearate, sorbitan monostearate, glyceryl monostearate, sorbitan monooleate, sorbitan trioleate, beeswax PEG-8 esters, and propylene glycol stearate, preferably the at least one first non-ionic surfactant (I1) is glycol distearate;
   the at least one second non-ionic surfactant (I2) is selected from the group consisting of cocamide monoethanolamine, cocamide diethanolamine, cocamide monoisopropanolamine, lauramide monoethanolamine, PEG-7 glyceryl cocoate, PEG-6 caprylic glycerides, lauryl glucoside, decyl glucoside, cetearyl glucoside, laureth-4, and PEG-8 caprylic glycerides, preferably the at least one second non-ionic surfactant (I2) are cocamide monoethanolamine and PEG-7 glyceryl cocoate;
   the at least one third non-ionic surfactant (I3) is selected from the group consisting of cetearyl alcohol, ceteareth-25, polysorbate 80, and PEG-40 hydrogenated castor oil, preferably the at least one third non-ionic surfactant (I3) is ceteareth-25; and
   the at least one anionic surfactant (I4) is selected from the group consisting of sodium laureth sulfate, sodium lauryl sulfate, ammonium lauryl sulfate, sodium stearate, sodium myreth sulfate and sodium coco-sulfate, preferably the at least one anionic surfactant (I4) is sodium laureth sulfate.
3. The mixture (I) according to embodiment 1 or 2, wherein the weight ratio of the total weight of the non-ionic surfactants to the total weight of the anionic surfactants is in the range from 3:1 to 8:1 [g/g].
4. The use of a mixture (I) according to any one of embodiments 1 to 3 for protecting the scalp during oxidative hair colouring and/or for providing high lightening during oxidative hair colouring.
5. An oxidative hair colouring chassis comprising a mixture (I) according to any one of embodiments 1 to 3, wherein the oxidative hair colouring chassis further comprises at least one of the following components:
   (F) at least one cosmetically acceptable aqueous or aqueous-organic medium and
   (H) at least one fatty substance.
6. The oxidative hair colouring chassis according to embodiment 5, wherein mixture (I) is present in an amount in the range of ≥ 0.1 wt.% to ≤ 30 wt.%, component (F) is present in an amount in the range of ≥ 30 wt.% to ≤ 90 wt.%, and component (H) is present in an amount in the range of ≥ 0.1 wt.% to ≤ 20 wt.%, each based on the total weight of the oxidative hair colouring chassis.
7. The oxidative hair colouring chassis according to embodiment 5 or 6, wherein the oxidative hair colouring chassis further comprises the following components
   (C) at least one chelant and
   (D) at least one alkalizing agent.
8. The oxidative hair colouring chassis according to any one of embodiments 5 to 7, wherein component (C) comprises diethylenetriamine penta(methylene phosphonic acid), and salts thereof.
9. The oxidative hair colouring chassis according to any one of embodiments 5 to 8, wherein component (D) is at least one alkalizing agent selected form the group consisting of 2-amino-1-propanol, 2-amino-2-methylpropan-1-ol and monoethanolamine, preferably monoethanolamine.
10. The oxidative hair colouring chassis according to any one of embodiments 5 to 9, wherein component (F) consists of water.
11. The oxidative hair colouring chassis according to any one of embodiments 5 to 10, wherein component (H) is selected from the group consisting of liquid hydrocarbons, non-silicone oils of animal, plant, mineral or synthetic origin, fatty alcohols, fatty acid esters and/or fatty alcohol esters, non-silicone waxes, and silicones or mixtures thereof.
12. The oxidative hair colouring chassis according to any one of embodiments 5 to 11, wherein the oxidative hair colouring chassis has a pH in the range of ≥ 8.5 to ≤ 11.5.
13. An oxidative hair colouring composition comprising an oxidative hair colouring chassis according to any one of embodiments 5 to 12 and the following components
   (A) at least one oxidative coupler dye precursor,
   (B) at least one oxidative primary dye precursor, and
   (E) at least one oxidizing agent.
14. The oxidative hair colouring composition according to embodiment 13, wherein component (A) is at least one oxidative coupler dye precursor selected from the group consisting of resorcinol, 4-chlororesorcinol, 2-chlororesorcinol, 2-methylresorcinol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, hydroxybenzomorpholine, 2-amino-5-ethylphenol, 6-amino-m-cresol, 6-amino-o-cresol, 2,4-diaminophenoxyethanol, 2-amino-4-hydroxyethylaminoanisole, 1,3-bis-(2,4-diaminophenoxy)propane, 2,6-dihydroxyethylaminotoluene, m-phenylenediamine, 2,4-diamino-1,5-di(2-hydroxyethoxybenzene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindole, 6-hydroxyindole, dihydroxyindoline, phenyl methyl pyrazolone, 1,2,4-trihydroxybenzene, 5-((2-hydroxyethyl)amino)-1,3-benzodioxol, isatin, hydroquinone, 4-formyl-1-methylquinolinium-p-toluenesulfonate and salts thereof.
15. The oxidative hair colouring composition according to embodiment 13 or 14, wherein component (B) is at least one oxidative primary dye precursor selected from the group consisting of toluene-2,5-diamine, p-phenylenediamine, n-phenyl-p-phenylenediamine, N,N-bis(2-hydroxyethyl)-p-phenylenediamine, hydroxyethyl-p-phenylenediamine sulphate, hydroxypropyl bis(n-hydroxyethyl-p-phenylenediamine), 2-methoxymethyl-p-phenylenediamine, 2-chloro-p-phenylenediamine, p-aminophenol, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diaminodihydroxypyrazolopyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and salts thereof.
16. The oxidative hair colouring composition according to any one of embodiments 13 to 15, wherein component (E) is hydrogen peroxide.
17. A kit comprising
   (i) a tint composition comprising components (A), (B), (C), (D) and a mixture (I) as defined in any one of the preceding embodiments, and
   (ii) an oxidative composition comprising component (E) as defined any in any one of the preceding embodiments.
18. A method of treating hair comprising the steps of:
   a. providing the tint composition (i) comprising components (A), (B), (C), (D) and a mixture (I) as defined in any one of the preceding embodiments,
   b. providing an oxidative composition (ii) comprising component (E) as defined in any one of the preceding embodiments,
   c. mixing the tint composition (i) and the oxidative composition (ii) to obtain a mixed oxidative hair colouring composition according to any one of the preceding embodiments,
   d. applying the oxidative hair colouring composition onto the hair;
   e. leaving the oxidative hair colouring composition on the hair for a period in the range from ≥ 5 to ≤ 50 minutes; and
   f. subsequently rinsing the oxidative hair colouring composition from the hair.

### EXAMPLES

The following examples illustrate the formulations and performance results according to the presently claimed invention. These examples are not intended to be inclusive of all aspects of the subject matter disclosed herein, but rather to illustrate representative compositions and results. These examples are not intended to exclude equivalents and variations of the presently claimed invention, which are apparent to one skilled in the art.

### Materials

Sodium diethylenetriamine pentamethylene phosphonate commercially available from Zschimmer & Schwarz.

Trisodium ethylenediamine disuccinate commercially available from Innospec.

Disodium EDTA commercially available from BASF.

Monoethanolamine, cosmetic grade (5 ppm DEA) available from INEOS Group.

Cetearyl Alcohol available from BASF.

Glycol Distearate available from Evonik.

Sodium laureth sulfate (SLE2S), 70% in water available from BASF.

Cocamide monoetanolamine available from BASF.

Cetiol HE (Peg-7 glyceryl cocoate) available from BASF.

CETEARETH-25 (C16-18 alcohols, ethoxylated) available from BASF.

Sodium sulfate, anhydrous available from Cordenka.

Sodium sulfite anhydrous available from BASF.

Ascorbic acid available from DSM Nutritional Products.

Citric acid anhydrous available from Cargill.

Polyquaternium-10 available from Dow Chemical.

### Method of Measurements

### 1. Measurement of the amount of any residual highly mobile aqueous phase via Rheology Filter Paper (RFP)

The residual highly mobile aqueous phase in an oxidative hair colouring composition was determined by Rheology Filter Paper (RFP) measurements.

15 g of the respective oxidative hair colouring chassis formulation and 15 g oxidative composition were mixed in a speedmixer for 5 minutes. In table 2, the respective oxidative hair colouring chassis formulations are shown, in table 3 the oxidative composition is shown.

2 g of the resulting mixture were added to the centre of a filter paper (125 mm diameter Whatman Grade 42 filter paper) with a syringe. The filter paper was taped on the plate of a Rheometer (TA-Instrument AR 2000ex Rheometer). A 60 mm-diameter silicone gasket was laid on the filter paper and the Rheometer was instructed to close the gap to 800 microns carefully ensuring the gasket fits around it and does not interfere with the lowering geometry. At 5 min after the Rheology method has finished the rheometer was instructed to raise the geometry. When it was fully raised the filter paper was carefully remove. A black biro pen was used to draw an outline around where the product has spread through the filter paper. The excess product was scraped off using a spatula and the diameter of the spread was measured at 4 separate points. From this, the average diameter and spreading area in mm² were calculated and subtracted from the initial area the product has covered initially.

### 2. TSR (Transient Sensory Response)

The TSR is temporary mild itching, tingling and burning sensations caused by hair colourants during the application process. It is measured by the subjective assessment of the sensation by panelist according to a defined rating protocol on a scale of 4. To ensure statistical significance, a panellist number of at least 20 is needed.

### Examples:

**Table 2: Chassis formulations**

| | | Composition 1 | Compositi on 1a | Compositi on 1b | Compositi on 1c | Compositi on 1d | Compositi on 1e | Compositi on 2* | Compositi on 3* | Compositi on 4* |
|---|---|---|---|---|---|---|---|---|---|---|
| Chelant | | | | | | | | | | |
| (Component (C)) | Sodium diethylenetriamine pentamethylene phosphonate | 2,00 | 2,00 | 2,00 | 2,00 | 2,00 | 2,00 | 2,00 | 2,00 | 2,00 |
| | Trisodium ethylenediamine disuccinate | 0,50 | 0,50 | 0,50 | 0,50 | 0,50 | 0,50 | 0,50 | 0,50 | 0,50 |
| | Disodium EDTA | 0,10 | 0,10 | 0,10 | 0,10 | 0,10 | 0,10 | 0,10 | 0,10 | 0,10 |

| Alkalizing agent | | | | | | | | | | |
|---|---|---|---|---|---|---|---|---|---|---|
| (Component (D)) | Monoethanolamine, cosmetic grade (5 ppm DEA) | 10,00 | 10,00 | 10,00 | 10,00 | 10,00 | 10,00 | - | 10,00 | 10,00 |
| | Ammonia solution, 25% in water | - | - | - | - | - | - | 7,50 | - | - |

| Carrier | | | | | | | | | | |
|---|---|---|---|---|---|---|---|---|---|---|
| (Component (F)) | DI water | q.s. | q.s. | q.s. | q.s. | q.s. | q.s. | q.s. | q.s. | q.s. |
| | Propylene glycol (Pharma or cosmetic grade) | - | - | - | - | - | - | 8,00 | 8,00 | - |
| Fatty substance | | | | | | | | | | |
| (Componen t (H)) | Cetearyl Alcohol | 8,00 | 8,00 | 8,00 | 8,00 | 8,00 | 8,00 | 8,39 | 5,59 | 19,41 |

| Surfactant | | | | | | | | | | |
|---|---|---|---|---|---|---|---|---|---|---|
| (Componen t (I)) | | | | | | | | | | |
| I1 | Glycol Distearate | 2,00 | - | - | - | - | - | - | - | 0,55 |
| | Sorbitan trioleate | - | 2,00 | - | - | - | - | - | - | - |
| | Propylene glycol stearate | - | - | 2,00 | - | - | - | - | - | - |
| | Glyceryl monostearate | - | - | - | 1,20 | - | - | - | - | 6,78 |
| | Sorbitan monooleate | - | - | - | - | 1,20 | - | - | - | - |
| | Sorbitan monostearate | - | - | - | - | - | 1,20 | - | - | - |
| I2 | Cetiol HE (Peg-7 glyceryl cocoate) | 4,00 | 4,00 | - | - | - | - | - | - | - |
| | Cocamide monoetanolamine | 8,00 | - | - | - | - | - | - | - | - |
| | Laureth-4 | - | 8,00 | - | - | - | - | - | - | - |
| | Dicetyl phosphate and ceteth-10 phosphate | - | - | - | - | - | - | 2,61 | 1,74 | - |
| | Lauryl glucoside | - | - | 11,00 | - | - | - | - | - | - |
| | Cetearyl glucoside | - | - | - | 10,00 | - | - | - | - | - |
| | Decyl glucoside | - | - | - | - | 12,00 | - | - | - | - |
| | PEG-8 caprylic glycerides | - | - | - | - | - | 12,00 | - | - | - |
| I3 | CETEARETH-25 (C16-18 alcohols, ethoxylated) | 4,00 | - | - | - | - | - | - | - | - |
| | Polysorbate 80 | - | 3,00 | - | - | 4,80 | - | - | - | - |
| | PEG-40 hydrogenated castor oil | - | | 4,50 | - | - | 3,40 | - | - | - |
| | Sodium cocoyl isethionate | - | - | - | - | - | - | - | - | 0,51 |
| | Cetearyl alcohol | - | - | - | 3,50 | - | - | - | - | - |
| I4 | Sodium laureth sulfate (SLE2S), 70% in water | 5,00 | - | - | - | - | - | - | - | 4,00 |
| | Sodium lauryl sulfate (high pH, 29% Bulk) | - | - | - | - | - | - | - | - | 6,76 |
| | Steareth-200 | - | - | - | - | - | - | 1,00 | 0,67 | - |
| | Sodium stearate | - | 3,20 | - | - | 2,60 | - | - | - | - |
| | Ammonium lauryl sulfate | - | - | 5,60 | - | - | 6,90 | - | - | - |
| | Sodium myreth sulfate | - | - | - | 4,60 | - | - | - | - | - |
| Thickener | | | | | | | | | | |
| (Componen t (J)) | XanthangGum | - | - | - | - | - | - | - | - | - |
| | Sodium sulfate, anhydrous | 0,50 | 0,50 | 0,50 | 0,50 | 0,50 | 0,50 | - | - | - |
| | Ammonium sulfate | - | - | - | - | - | - | - | - | - |
| Antioxidant | | | | | | | | | | |
| | Sodium sulfite anhydrous | 0,40 | 0,40 | 0,40 | 0,40 | 0,40 | 0,40 | - | - | - |
| | Ascorbic acid | 0,30 | 0,30 | 0,30 | 0,30 | 0,30 | 0,30 | - | - | - |
| pH adjuster | | | | | | | | | | |
| | Citric acid anhydrous | 1,20 | 1,20 | 1,20 | 1,20 | 1,20 | 1,20 | - | - | - |
| Care product | | | | | | | | | | |
| | Polyquaternium-10 | 0,30 | 0,30 | 0,30 | 0,30 | 0,30 | 0,30 | - | - | - |

| Fragrance | | | | | | | | | | |
|---|---|---|---|---|---|---|---|---|---|---|
| | DIAMOND SPARK ES273997/00 | - | - | - | - | - | - | - | - | 0,30 |

| | | | | | | | | | | |
|---|---|---|---|---|---|---|---|---|---|---|
| q.s. = quantum satis *not within the scope of the invention | | | | | | | | | | |

**Table 3: Oxidative Composition (Developer 6%)**

| **Ingredients** | **6% Developer** |
|---|---|
| DI water | q.s. |
| Salicylic acid | 0.100 |
| Disodium phosphate | 0.080 |
| Phosphoric acid (85%) | 0.060 |
| Etidronic acid | 0.010 |
| Hydrogen peroxide (50% Hydrogen peroxide Interox co-SO, SOLVAY) | 12.000 |
| **Crème pre-mix formula** | **20.000** |
| Cetearyl alcohol (BASF) | (5.000) |
| Steareth-20 (CRODA) | (1.500) |
| DI water | (13.500) |

| | |
|---|---|
| q.s. = quantum satis | |

The formulations were prepared according to the following methods.

Crème pre-mix formula was a part of the oxidative formulation. A crème pre-mix is created by combining together DI water (68.5%), cetearyl alcohol (25%) and Steareth-20 (6.5%). The mixture was heated to 80 to 85 °C to melt the waxes and surfactant with stirring. The composition was homogenized for 5 minutes and then cooling was started to reduce the product to room temperature. The resulting crème pre-mix was thick and used within the oxidative composition.

### Chassis formulations:

The components of the chassis formulations for each composition were mixed at room temperate with DI water.

### Oxidative composition:

The components were mixed with the DI water at room temperature. When all the materials were dissolved, 20 wt.% of the crème pre-mix was added, and the mixture stirred until a uniform consistency was obtained. The product was then stored in plastic bottles until used.

**Table 4: Results**

| **Example** | **RFP [mm²]** | **TSR** |
|---|---|---|
| Composition 1 | 23 | 1.13 |
| Composition 1a | 43 | 1.22 |
| Composition 1b | 15 | 1.21 |
| Composition 1c | 47 | 1.15 |
| Composition 1d | 36 | 1.32 |
| Composition 1e | 37 | 1.18 |
| Composition 2* | 284 | 1 |
| Composition 3* | 61 | - |
| Composition 4* | 265 | 1.26 |

As can be seen from table 4, the inventive mixture (I) comprising the at least four surfactants (I1) to (I4) in the specified range reduces the amount of any residual highly mobile aqueous phase of the chassis formulation. By consequence the oxidative hair colouring chassis and/or composition of the presently claimed invention leads to a protection of the scalp during oxidative hair colouring as indicated by the low TSR. Additionally, a small halo (low RFP), i.e. low amounts of mobile aqueous phase, corelates with low TSR within a given chassis. High levels of mobile aqueous phase can negatively impact product performance, increase the risk of transient sensory response (TSR) during application, and reduce shelf-life stability.

Accordingly, the data includes six distinct formulations (Composition 1 through 1e), each incorporating variations of surfactant groups I1 to I4. Despite these variations, all six compositions consistently exhibit low RFP values-indicating high formulation stability-and low TSR values, which reflect reduced transient sensory responses during application. This reproducibility across multiple formulations strongly supports the credibility of the observed effect, i.e. the oxidative hair colouring chassis and/or composition of the presently claimed invention leads to a protection of the scalp during oxidative hair colouring. Moreover, the surfactant system is designed based on the Hydrophilic-Lipophilic Balance (HLB), a well-established and scientifically validated parameter used in cosmetic and pharmaceutical formulation science. The present invention surprisingly found that the HLB concept allows formulators to predict and control emulsion behavior, stability, and interfacial activity, while the use of multiple surfactants with complementary HLB values enables the creation of a synergistic system that minimizes the presence of free water and enhances emulsion stability.

The absence of molecular weight considerations is right, since it was further found that the molecular weight is not the primary determinant of emulsion stability nor aqueous phase mobility. Instead, the inventors surprisingly found that the key factors are the surfactants' interfacial properties, their HLB values, and their ability to form structured emulsions. The experimental shows clearly shows that formulations lacking the defined surfactant system (e.g., Compositions 2*, 3*, and 4*) exhibit significantly higher RFP values and, in some cases, elevated TSR values. This contrast further substantiates the functional relevance of the surfactant combination and the validity of the HLB-based design.

Finally, the practical implications of the inventive compositions-namely, improved scalp protection and reduced irritation during oxidative hair coloring-underscore the formulation's effectiveness. These benefits are directly linked to the reduction of mobile aqueous phase and the optimized surfactant system, making the generalization not only credible but also functionally justified.

## Claims

1. A mixture (I) comprising at least four surfactants (I1) to (I4), wherein the mixture (I) comprises
(I1) 5 to 15 wt.% of at least one first non-ionic surfactant having an HLB value in the range of 1 to 9,
(I2) 45 to 65 wt.% of at least one second non-ionic surfactant having an HLB value in the range of higher than 9 to 14,
(I3) 10 to 30 wt.% of at least one third non-ionic surfactant having an HLB value in the range of higher than 14 to 20,
(I4) 10 to 30 wt.% of at least one anionic surfactant having an HLB value of higher than 17,
wherein the % by weight values in each case are based on the total weight of the surfactants (I1), (I2), (I3) and (I4).

2. The mixture (I) according to claim 1, wherein
the at least one first non-ionic surfactant (I1) is selected from the group consisting of glycol distearate, sorbitan monostearate, glyceryl monostearate, sorbitan monooleate, sorbitan trioleate, beeswax PEG-8 esters, and propylene glycol stearate, preferably the at least one first non-ionic surfactant (I1) is glycol distearate;
the at least one second non-ionic surfactant (I2) is selected from the group consisting of cocamide monoethanolamine, cocamide diethanolamine, cocamide monoisopropanolamine, lauramide monoethanolamine, PEG-7 glyceryl cocoate, PEG-6 caprylic glycerides, lauryl glucoside, decyl glucoside, cetearyl glucoside, laureth-4, and PEG-8 caprylic glycerides, preferably the at least one second non-ionic surfactant (I2) are cocamide monoethanolamine and PEG-7 glyceryl cocoate;
the at least one third non-ionic surfactant (I3) is selected from the group consisting of cetearyl alcohol, ceteareth-25, polysorbate 80, and PEG-40 hydrogenated castor oil, preferably the at least one third non-ionic surfactant (I3) is ceteareth-25; and
the at least one anionic surfactant (I4) is selected from the group consisting of sodium laureth sulfate, sodium lauryl sulfate, ammonium lauryl sulfate, sodium stearate, sodium myreth sulfate and sodium coco-sulfate, preferably the at least one anionic surfactant (I4) is sodium laureth sulfate.

3. The mixture (I) according to claim 1 or 2, wherein the weight ratio of the total weight of the non-ionic surfactants to the total weight of the anionic surfactants is in the range from 3:1 to 8:1 [g/g].

4. The use of a mixture (I) according to any one of claims 1 to 3 for protecting the scalp during oxidative hair colouring and/or for providing high lightening during oxidative hair colouring.

5. An oxidative hair colouring chassis comprising a mixture (I) according to any one of claims 1 to 3, wherein the oxidative hair colouring chassis further comprises at least one of the following components:
(F) at least one cosmetically acceptable aqueous or aqueous-organic medium and
(H) at least one fatty substance.

6. The oxidative hair colouring chassis according to claim 5, wherein mixture (I) is present in an amount in the range of ≥ 0.1 wt.% to ≤ 30 wt.%, component (F) is present in an amount in the range of ≥ 30 wt.% to ≤ 90 wt.%, and component (H) is present in an amount in the range of ≥ 0.1 wt.% to ≤ 20 wt.%, each based on the total weight of the oxidative hair colouring chassis.

7. The oxidative hair colouring chassis according to claim 5 or 6, wherein the oxidative hair colouring chassis further comprises the following components
(C) at least one chelant and
(D) at least one alkalizing agent.

8. The oxidative hair colouring chassis according to any one of claims 5 to 7, wherein component (C) comprises diethylenetriamine penta(methylene phosphonic acid), and salts thereof.

9. The oxidative hair colouring chassis according to any one of claims 5 to 8, wherein component (D) is at least one alkalizing agent selected form the group consisting of 2-amino-1-propanol, 2-amino-2-methylpropan-1-ol and monoethanolamine, preferably monoethanolamine.

10. The oxidative hair colouring chassis according to any one of claims 5 to 9, wherein component (F) consists of water.

11. The oxidative hair colouring chassis according to any one of claims 5 to 10, wherein component (H) is selected from the group consisting of liquid hydrocarbons, non-silicone oils of animal, plant, mineral or synthetic origin, fatty alcohols, fatty acid esters and/or fatty alcohol esters, non-silicone waxes, and silicones or mixtures thereof.

12. The oxidative hair colouring chassis according to any one of claims 5 to 11, wherein the oxidative hair colouring chassis has a pH in the range of ≥ 8.5 to ≤ 11.5.

13. An oxidative hair colouring composition comprising an oxidative hair colouring chassis according to any one of claims 5 to 12 and the following components
(A) at least one oxidative coupler dye precursor,
(B) at least one oxidative primary dye precursor, and
(E) at least one oxidizing agent, preferably wherein component (A) is at least one oxidative coupler dye precursor selected from the group consisting of resorcinol, 4-chlororesorcinol, 2-chlororesorcinol, 2-methylresorcinol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, hydroxybenzomorpholine, 2-amino-5-ethylphenol, 6-amino-m-cresol, 6-amino-o-cresol, 2,4-diaminophenoxyethanol, 2-amino-4-hydroxyethylaminoanisole, 1,3-bis-(2,4-diaminophenoxy)propane, 2,6-dihydroxyethylaminotoluene, m-phenylenediamine, 2,4-diamino-1,5-di(2-hydroxyethoxybenzene, 1-naphthol, 2-methyl-1-naphthol, 1,5-naphthalenediol, 2,7-naphthalenediol, 1-acetoxy-2-methylnaphthalene, 2,6-dihydroxy-3,4-dimethylpyridine, 2,6-dimethoxy-3,5-pyridinediamine, 3-amino-6-methoxy-2-(methylamino)-pyridine, 2-amino-3-hydroxypyridine, 2,6-diaminopyridine, dihydroxyindole, 6-hydroxyindole, dihydroxyindoline, phenyl methyl pyrazolone, 1,2,4-trihydroxybenzene, 5-((2-hydroxyethyl)amino)-1,3-benzodioxol, isatin, hydroquinone, 4-formyl-1-methylquinolinium-p-toluenesulfonate and salts thereof, and/or wherein component (B) is at least one oxidative primary dye precursor selected from the group consisting of toluene-2,5-diamine, p-phenylenediamine, n-phenyl-p-phenylenediamine, N,N-bis(2-hydroxyethyl)-p-phenylenediamine, hydroxyethyl-p-phenylenediamine sulphate, hydroxypropyl bis(n-hydroxyethyl-p-phenylenediamine), 2-methoxymethyl-p-phenylenediamine, 2-chloro-p-phenylenediamine, p-aminophenol, p-methylaminophenol, 4-amino-m-cresol, 6-amino-m-cresol, bis(5-amino-2-hydroxyphenyl)methane, tetraaminopyrimidine, 2,5,6-triamino-4-pyrimidinol, 4,5-diamino-1-(2-hydroxyethyl)pyrazol 2,3-diaminodihydroxypyrazolopyrazolone dimethosulfonate, 4,5-diamino-1-hexylpyrazol, hydroxypropyl-p-phenylenediamine, dimethylpiperazinium aminopyrazolopyridine chloride hydrochloride, methylimidazoliumpropyl p-phenylenediamine, hydroxyethoxy aminopyrazolopyridine and salts thereof, and/or wherein component (E) is hydrogen peroxide.

14. A kit comprising
(i) a tint composition comprising components (A), (B), (C), (D) and a mixture (I) as defined in any one of the preceding claims, and
(ii) an oxidative composition comprising component (E) as defined any in any one of the preceding claims.

15. A method of treating hair comprising the steps of:
a. providing the tint composition (i) comprising components (A), (B), (C), (D) and a mixture (I) as defined in any one of the preceding claims,
b. providing an oxidative composition (ii) comprising component (E) as defined in any one of the preceding claims,
c. mixing the tint composition (i) and the oxidative composition (ii) to obtain a mixed oxidative hair colouring composition according to any one of the preceding claims,
d. applying the oxidative hair colouring composition onto the hair;
e. leaving the oxidative hair colouring composition on the hair for a period in the range from ≥ 5 to ≤ 50 minutes; and
f. subsequently rinsing the oxidative hair colouring composition from the hair.
